# EUROPÄISCHE PATENTSCHRIFT

(11) **EP 2 741 700 B1**
(45) Veröffentlichungstag und Bekanntmachung des Hinweises auf die Patenterteilung: **11.10.2017**
(21) Anmeldenummer: 12746322.2
(22) Anmeldetag: 08.08.2012
(51) Int. Cl.: A61B 17/86, A61C 8/00, A61C 3/00, A61F 2/28, A61L 27/00, A61L 27/46, A61L 27/56

(54) **KÖRPER AUS KNOCHENERSATZMATERIAL UND VERFAHREN ZUR HERSTELLUNG**
BODY MADE OF BONE SUBSTITUTE MATERIAL AND METHOD FOR PRODUCTION
CORPS EN MATÉRIAU DE REMPLACEMENT OSSEUX ET PROCÉDÉ DE FABRICATION

(30) Priorität: 11.08.2011 CH 13302011
(43) Veröffentlichungstag der Anmeldung: 18.06.2014
(73) Patentinhaber: regenHu AG, 1690 Villaz-St-Pierre (CH)
(72) Erfinder: THURNER, Marc, 2068 Hauterive (CH); KUSTER, Michael, 3012 Bern (CH)
(74) Vertreter: Rentsch Partner AG
(86) Internationale Anmeldenummer: PCT/EP2012/065514
(87) Internationale Veröffentlichungsnummer: WO 2013/021000

(56) Entgegenhaltungen:
- EP-A2- 1 277 450
- WO-A1-2007/027794
- WO-A1-2011/030185
- WO-A2-2006/127392
- US-B1- 6 214 049

## Beschreibung

### TECHNISCHES GEBIET

Die Erfindung betrifft einen biokompatiblen Körper zur Implantation in eine Kavität eines Gewebes, z.B. Knochen, umfassend eine Grundstruktur aus einem Knochenersatzmaterial. Die Erfindung betrifft weiter ein Verfahren zur Herstellung eines biokompatiblen Körpers zur Implantation in eine Kavität eines Gewebes, z.B. Knochen.

### STAND DER TECHNIK

Synthetisches oder natürliches Knochenersatzmaterial wird in der Medizin, insbesondere in der Zahnmedizin bei der Zahnimplantation, verwendet, um Defekte oder Kavitäten im Knochen aufzufüllen. Die Materialien sind porös und oft mit Substanzen angereichert, welche die Knochenneubildung fördern (osteinduktive Substanzen). Bei Personen, welche unter Kieferknochenschwund infolge eines Zahnverlusts oder einer Parodontitis leiden, ist für ein Zahnimplantat meist nicht genügend Knochen vorhanden, weshalb vor der Versorgung mit einem Zahnimplantat aufwendige Knochenaufbau-Massnahmen erforderlich sind. Daher sind für die Versorgung oft mehrere chirurgische Operationen notwendig. In einer ersten chirurgischen Operation wird ein Knochenersatzmaterial in eine Kavität im Kieferknochen oder in den Bereichen, wo aufgrund des Knochenschwunds ungenügend Knochen vorhanden ist, eingebracht. Damit eine ungestörte Regeneration ermöglicht wird, muss das eingebrachte Knochenersatzmaterial oft in einer komplizierten und schwierigen Operation mit einer Membran (z.B. eine Bio-Gide® Membrane der Firma Geistlich AG) geschützt werden. Die Membran verhindert unter anderem das Einwachsen von Weichgewebe in die mit Knochenersatzmaterial gefüllte Kavität. Anschliessend wird das eingebrachte Knochenersatzmaterial während einer Regenerationszeit von mehreren Monaten nach und nach durch neugebildeten Knochen durchbaut bzw. ersetzt (Osteokonduktion). Nach der Regenerationszeit wird in einer zweiten chirurgischen Operation ein Implantatkörper in den neugebildeten Knochen eingesetzt, auf welches später in einem weiteren Schritt ein Abutment oder direkt eine Krone, eine Brücke oder eine andere Prothetik aufgesetzt wird. Chirurgische Operationen sind teuer und es besteht immer ein Infektionsrisiko. Zudem führt das Einsetzen eines Implantats zu post-operativen Schmerzen am Ort der Implantation. Auch ist die Lebensqualität des Patienten während der Regenerationszeit stark beeinträchtigen. Beispielsweise betragen die Regenerationszeiten zwischen den zwei chirurgischen Operationen bei einer Zahnimplantation typischerweise drei bis sechs Monate.

### DARSTELLUNG DER ERFINDUNG

Aufgabe der Erfindung ist es, einen biokompatiblen Körper der eingangs genannten Art zur vereinfachten Implantation in eine Kavität eines Knochens bereitzustellen, welcher, z.B. bei einer Zahnimplantation, die Behandlungskosten und das Infektionsrisiko beim Patienten verringert. Zudem soll er die Lebensqualität des Patienten während der gesamten Behandlung erhöhen.

Diese Aufgabe wird durch die Merkmale des Anspruch 1 gelöst. Der erfindungsgemässe biokompatible Körper der eingangs genannten Art ist demnach dadurch gekennzeichnet, dass der Körper ein Anschlusselement aufweist, wobei das Anschlusselement zumindest teilweise in der Grundstruktur des Körpers eingebettet ist.

In einer einfachen chirurgischen Operation kann der erfindungsgemässe Körper in eine Kavität im Knochen eingesetzt werden, wobei die Grundstruktur des Köpers die Kavität oder den Bereichen, wo aufgrund des Knochenschwunds ungenügend Knochen vorhanden ist, ausfüllt. Anschliessend wird die Grundstruktur aus Knochenersatzmaterial des eingebrachten Körpers während einer Regenerationszeit von mehreren Monaten nach und nach durch neugebildeten Knochen durchbaut bzw. ersetzt. Nach der Regenerationszeit kann an dem Anschlusselement z.B. ein Abutment oder direkt eine Krone, eine Brücke oder eine andere Prothetik angebracht werden. Eine komplizierte und schwierige Operation zum Anbringen einer Membran und/oder eine weitere chirurgische Operation zum Einsetzen eines Implantatkörpers in den neugebildeten Knochen ist nicht mehr notwendig. Der erfindungsgemässe biokompatible Körper ermöglicht es demnach, Behandlungen, welche oft zwei chirurgische Operationen benötigten, und dadurch zu einem höheren Infektionsrisiko, zu höheren Behandlungskosten, post-operativen Schmerz am Implantationsort, und einer schlechten Lebensqualität wegen der langen Zeitdauer zwischen den zwei Operationen führen, mit einer einzigen chirurgischen Operation durchzuführen. Dadurch können die Kosten und das Infektionsrisiko gesenkt werden und die lange Wartezeit zwischen den Operationen kann vermieden werden.

In einer ersten bevorzugten Ausführungsform des erfindungsgemässen biokompatiblen Körpers weist das Anschlussstück einen Kopf auf, welcher derart in die Grundstruktur des Körpers eingebettet ist, dass die Grundstruktur den Kopf des Anschlussstücks kragenförmig umschliesst. Das Anschlussstück kann z.B. ein herkömmliches Zahnimplantat, eine Schraube oder ein speziell für die Einbettung hergestelltes Anschlussstück sein. Das Anschlussstück kann auch einen Verankerungsabschnitt aufweisen, mit welchem es in dem bestehenden Knochen eingesetzt werden kann, wobei die Grundstruktur des Körpers die Kavität im Knochen oder den Bereichen, wo aufgrund des Knochenschwunds ungenügend Knochen vorhanden ist, ausfüllt. Erfindungsgemäss weist das Anschlusselement einen Verankerungsabschnitt auf, wobei das Anschlusselement mit dem Verankerungsabschnitt formschlüssig in der Grundstruktur des Körpers eingebettet ist. Das Anschlussstück kann z.B. ein herkömmliches Zahnimplantat oder ein speziell für die Einbettung hergestelltes Anschlussstück sein. In dieser Ausführungsform kann der biokompatible Körper in eine Kavität des Knochens eingesetzt werden.

Indem das Anschlussstück in die Grundstruktur aus Knochenersatzmaterial eingebettet ist, resp. formschlüssig von dem Knochenersatzmaterial umschlossen wird, ist eine grössere Formvielfalt für den Verankerungsabschnittes ermöglicht. Es ist nicht notwendig, dass der Verankerungsabschnitt eine Form aufweist, mit welcher er in eine Grundstruktur eingeschlagen oder eingeschraubt werden kann. Das Anschlussstück muss auch nicht in der Grundstruktur festgeleimt oder -zementiert werden.

Unter Knochenersatzmaterial wird ein Material verstanden, welches zur Auffüllung von Knochendefekten oder grösseren Kavitäten im Knochen, z.B. bei einer Zahnimplantation, verwendet wird. Es soll ein Durchwachsen der Kavität durch Weichgewebe verhindern und den Knochaufbau begünstigen. Dabei kann der neugebildete Knochen mit dem Knochenersatzmaterial zusammenwachsen oder das Knochenersatzmaterial kann teilweise oder ganz abgebaut und durch den neugebildeten Knochen ersetzt werden. Das Knochenersatzmaterial kann synthetisch oder natürlich sein.

Die Grundstruktur des Körpers ist vorzugsweise aus einem formstabilen aber leicht bearbeitbaren, Knochenersatzmaterial gefertigt, so dass die Form des Körpers vor dem Einsetzten in die Kavität des Knochens oder den Bereichen, wo aufgrund des Knochenschwunds ungenügend Knochen vorhanden ist, an die Form der Kavität oder den Bereich angepasst werden kann. Die Grundstruktur kann auch direkt so geformt sein, dass sie in die Kavität oder den Bereich hineinpasst.

In allen Ausführungsformen weist das Anschlusselement vozugsweise eine Aufnahmeöffnung für ein Verbindungs- oder Abschlusselement auf. Die Aufnahmeöffnung ist von ausserhalb der Grundstruktur des Körpers für das Verbindungs- oder Abschlusselement zugänglich. Sie kann aber auch in der Grundstruktur des Körpers liegen und erst vor dem Einsetzen des Körpers in die Kavität des Knochens freigelegt werden. Zudem kann sie mit einem Verschlusselement, z.B. ein Deckel, verschlossen sein, welches vor dem Anbringen des Verbindungs- oder Abschlusselements entfernt werden kann. Erfindungsgemäss ist der Verankerungsabschnitt derart geformt, dass er verdrehsicher in der Grundstruktur eingebettet ist. Die kann z.B. durch einen nicht-kreisförmigen Querschnitt des Verankerungsabschnitts erreicht werden. Eine solche verdrehsichere Form des Verankerungsabschnitts weist z.B. einen rechteckigen Querschnitt auf oder es sind Rippen, Flügel, Flossen, Noppen, Haken, Vorsprünge, Plättchen, Gitter oder dergleichen ausgebildet.

Zusätzlich zur verdrehsicheren Form oder unabhängig davon, weist der Verankerungsabschnitt des erfindungsgemässen Körpers vorzugsweise einen Hinterschnitt auf, mit welchem er in der Grundstruktur in Eingriff ist, so dass der Verankerungsabschnitt nicht ohne Beschädigung der Grundstruktur aus dieser herausgezogen werden kann. Dies kann z.B. durch unterschiedlich grosse Querschnitte des Verankerungselements erreicht werden oder durch Aussparungen im Anschlusselement, in welche die Grundstruktur eingreifen kann.

In allen Ausführungsformen kann das Anschlusselement einstückig oder mehrstückig ausgebildet sein. Das Anschlusselement ist vorzugsweise aus Keramik, Metall, Polymer oder Plastik gefertigt. Zudem kann die Grundstruktur zur Verbesserung der Knochenneubildung osteokonduktiv sein. Dies kann z.B. durch Hohlräume oder Poren erreicht werden, welche zusätzlich mit osteoinduktiven Zusammensetzungen und/oder pharmazeutischen Produkten angereichert sein können. In den Hohlräumen können auch weitere bioaktive Stoffe mit unterschiedlichen Funktionen eingelagert werden, wie z.B. Stoffe die das Einwachsen von Weichgewebe in die Grundstruktur verhindern.

Von osteoinduktiv (osseoinduktiv) spricht man, wenn eine Substanz in der Lage ist die Knochenneubildung anzuregen. Wenn ein Material in der Lage ist, als Leitgerüst das natürliche Knochenwachstum zu erleichtern, spricht man von Osteokonduktion. Ein solches Material ist osteokonduktiv oder weist eine osteokonduktive Struktur auf.

Vorzugsweise weist die Grundstruktur eine makroporöse, interkonektierte Struktur auf. Die Grundstruktur kann z.B. eine Gyroid- oder gyroid-ähnliche Struktur sein.

Weiter kann die Grundstruktur an den Seiten, welche im implantieren Zustand dem Weichgewebe zugewandt sind, eine geschlossene Struktur aufweisen, um beispielsweise bei dentalen Anwendungen ein Einwachsen von Weichgewebe und eine damit einhergehende Reduzierung des knöchernen Umbaus zu vermeiden. Eine Membran, wie in anderen herkömmlichen dentalen Anwendungen empfohlen, ist daher nicht notwendig.

Vorzugsweise umfasst das Knochenersatzmaterial als strukturgebende Zusammensetzung ein Material, ausgewählt aus der Gruppe Calcium Phosphat, vorzugweise Calcium Hydroxyapaptite (Ca₁₀(PO₄)₆(OH)₂, HA), beta-Tricalciumphosphat (Ca₃(PO₄)₂, beta-TCP) oder Biphasisches Calciumphosphat; Bioglas; Biopoylmer, vorzugsweise Polymilchsäure oder Polylactid (PLA), Poly-L-lactide (PLLA), Chitosan, Collagen, Alginate, Silikon, synthetische Hydrogels oder Hyaluronan; und Mischungen davon.

Als osteoinduktive Zusammensetzungen könne z.B. Knochenmark Extrakt, pallet-rich-Plasma (PRP), Trombocytenmediator-konzentrat (TMK) oder synthetische Faktoren wie rhBMP (recombinant humen bone mophogenetic proteins) verwendet werden.

In einer weiteren Ausführungsform weist der biokompatible Körper zusätzlich zur Grundstruktur aus einem Knochenersatzmaterial eine zweite Grundstruktur aus einem biokompatiblen Material, vorzugsweise ein Knorpelersatzmaterial oder Ersatz-Weichgewebe, z.B. ein Ersatz-Hautgewebe, auf. Das biokompatible Material kann ein Material basierend auf Kollagen sein. Die zweite Grundstruktur kann auf oder in der Grundstruktur aus Knochenersatzmaterial angeordnet sein und wird mit demselben Verfahren, aber mit anderen Materialien, aufgebaut.

Die Erfindung betrifft weiter ein Verfahren zur Herstellung eines erfindungsgemässen biokompatiblen Körpers zur Implantation in eine Kavität eines Knochens. Das Verfahren umfasst folgende Schritte: (i) Bereitstellen eines Anschlusselements; und (ii) Anbringen einer Grundstruktur aus Knochenersatzmaterial, so dass das Anschlusselement in der Grundstruktur aus Knochenersatzmaterial eingebettet ist.

Vorzugsweise wird zur Herstellung des erfindungsgemässen biokompatiblen Körpers zur Implantation in eine Kavität eines Knochens ein Verfahren zur Herstellung von Blöcken aus Knochersatzmaterial verwendet, wie es in WO 2009/040352 beschrieben ist. Bei dem Verfahren wird eine Grundstruktur aus Knochenersatzmaterial in mehreren Schichten übereinander aufgebaut wird. Die Schichten umfassen jeweils mehrere, voneinander beabstandete, vorzugsweise parallel zueinander verlaufende, Linien aus Knochenersatzmaterial. In den Zwischenräumen zwischen den Linien einer Schicht kann zusätzlich eine osteoinduktive Zusammensetzung eingebracht werden. Mit dem Verfahren kann ein Knochenersatzmaterial in 3-dimensionalen Blöcken mit der gewünschten Form hergestellt werden oder ein Verbundmaterial (Composite), welches aus mehreren Materialien besteht. Diese weisen eine makroporöse, interkonektierte Struktur auf. In dieser Hinsicht wird Bezug genommen auf die Anmeldung WO 2009/040352. Mit dem Verfahren können auch Körper mit beliebiger äusseren und inneren Struktur hergestellt werden, indem das Material in Form von einzelnen Punkten oder kurzen Linien Schicht um Schicht aufgetragen wird. Beispielsweise ermöglicht das Verfahren die Herstellung einer Gyroid- oder gyroid-ähnlichen Struktur. Bei dem bevorzugten Herstellungsverfahren wird eine Grundstruktur aus Knochenersatzmaterial Schicht für Schicht um das Anschlusselement aufgebaut, bis dieser zumindest teilweise in der Grundstruktur eingebettet ist. Dabei können auch verdrehsichere Elemente und Hinterschnitte des Verankerungsabschnitts vollständig eingebettet, resp. von der Grundstruktur umschlossen werden. Die Linien der anzubringenden Schicht stehen vorzugsweise in einem Winkel, vorzugsweise von etwa 30 bis 90 Grad, zu den Linien der vorangehenden Schicht, so dass eine makroporöse, interkonektierte Struktur entsteht, welche mit osteoinduktiven Zusammensetzungen und/oder pharmazeutischen oder bioaktiven Produkten gefüllt werden kann. Ein Körper, welcher eine solche makroporöse, interkonektierte Struktur aufweist, kann z.B. durch annähen an einem Knochen befestigt werden. Die Schichten müssen jedoch nicht als durchgezogenen Linien in einem bestimmten Winkel zueinander aufgetragen werden. Die Schichten können in beliebiger Weise nacheinander aufgetragen werden, so dass auch andere Strukturen, beispielsweise die oben genannten Gyroid- oder gyroid-ähnlichen Strukturen, hergestellt werden können.

In einer weiteren Ausführungsform des erfindungsgemässen Verfahrens, kann die Grundstruktur aus Knochenersatzmaterial um den Verankerungsabschnitt gegossen oder aufgeschäumt werden.

Der erfindungsgemässe biokompatible Körper mit dem Anschlusselement kann vorzugsweise in der Zahnmedizin bei der Zahnimplantation verwendet werden. Eine Verwendung als Anschluss für ein künstliches Gelenk, z.B. Fingergelenk, ist ebenfalls möglich.

### KURZE ERLÄUTERUNG ZU DEN FIGUREN

Die Erfindung soll nachfolgend anhand von Ausführungsbeispielen im Zusammenhang mit der Zeichnung näher erläutert werden. Es zeigen:
- Fig. 1: eine Ausführungsform eines erfindungsgemässen Körpers mit eingebettetem Anschlusselement unter (a) in einer Seitenansicht mit Schnitt durch den Kopf entlang der Schnittlinie C-C aus Fig.1(b) und unter (b) in einer Draufsicht;
- Fig. 2: eine perspektivische Ansicht einer Ausführungsform eines erfindungsgemässen Körpers mit eingebettetem Anschlusselement;
- Fig. 3: einen Schnitt entlang einer Linie A-A wie in Fig. 2 durch eine Ausführungsform eines erfindungsgemässen Körpers mit eingebettetem Anschlusselement;
- Fig. 4: einen Schnitt entlang einer Linie B-B wie in Fig. 2 durch eine Ausführungsform eines erfindungsgemässen Körpers mit eingebettetem Anschlusselement;
- Fig. 5: eine Ausführungsform eines Anschlusselement unter (a) in einer Seitenansicht und unter (b) in einer Unteransicht;
- Fig. 6: eine weitere Ausführungsform eines Anschlusselement unter (a) in einer Seitenansicht und unter (b) in einer Unteransicht;
- Fig. 7: eine weitere Ausführungsform eines Anschlusselement unter (a) in einer Seitenansicht und unter (b) in einer Unteransicht;
- Fig. 8: eine schematische Darstellung eines eingenähten erfindungsgemässen Körpers; und
- Fig. 9: eine weitere Ausführungsform eines erfindungsgemässen Körpers mit eingebettetem Anschlusselement.

### WEGE ZUR AUSFÜHRUNG DER ERFINDUNG

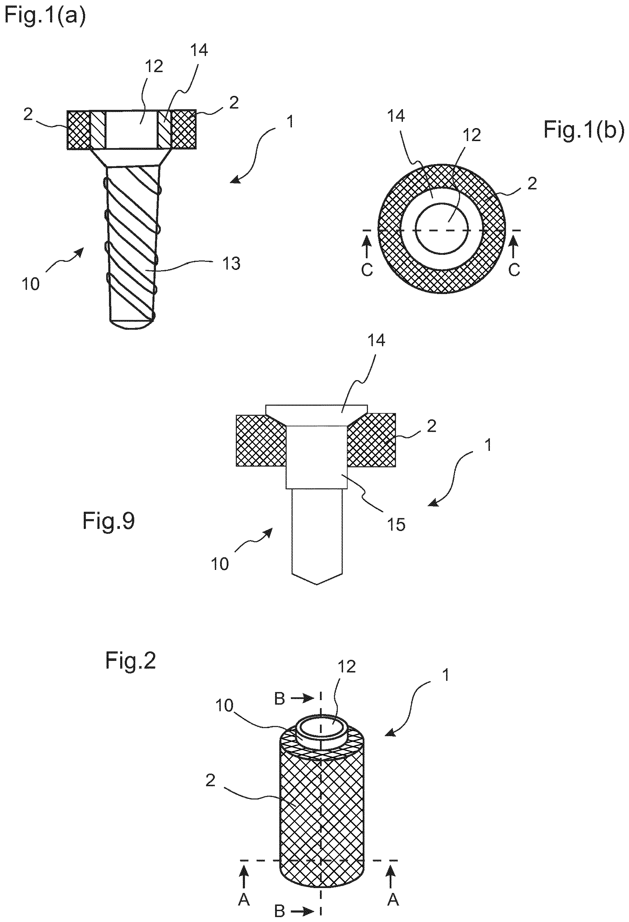

Fig. 1 zeigt eine Ausführungsform eines erfindungsgemässen Körpers 1 mit eingebettetem Anschlusselement 10. Unter (a) ist der Körper in einer Seitenansicht mit Schnitt durch einen Kopf 14 des Anschlusselements 10 entlang der Schnittlinie C-C aus Fig.1(b) dargestellt. Unter (b) ist der Körper 1 in einer Draufsicht auf eine Aufnahmeöffnung 12 des Anschlusselements 10 dargestellt. Das Anschlusselement ist z.B. ein herkömmliches Zahnimplantat, welches einen als "Schraube" ausgebildeten Verankerungsabschnitt aufweist. Der Körper 1 umfasst weitere eine Grundstruktur 2 aus einem biokompatiblen Knochenersatzmaterial, in welche der Kopf 14 des Anschlusselements 10 eingebettet ist, so dass die Grundstruktur 2 den Kopf 14 kragenförmig umschliesst. Die Grundstruktur 2 ist als zylindrischer Körper dargestellt, kann aber je nach der Form der Kavität, in welche der Körper eingesetzt werden soll, auch andere Formen aufweisen. Zudem kann die Grundstruktur aus einem formstabilen aber leicht bearbeitbarem Material gefertigt sein, so dass er vor dem Einsetzen leicht an die Form der Kavität angepasst werden kann. Weiter Eigenschaften der Grundstruktur sind weiter unten beschrieben.

Fig. 9 zeigt eine weitere Ausführungsform eines erfindungsgemässen Körpers 1 mit eingebettetem Anschlusselement 10. Im Unterschied zur Ausführungsform in Fig.1(a) ist das Anschlusselement eine Schraube, z.B. eine Senkschraube, und mit einem Kopfbereich 14 und/oder Halsbereich 15 der Schraube teilweise in die Grundstruktur 2 eingebettet.

Fig. 2 zeigt eine perspektivische Ansicht einer Ausführungsform eines erfindungsgemässen Körpers 1 mit eingebettetem Anschlusselement 10. Der Körper 1 umfasste eine Grundstruktur 2 aus einem biokompatiblen Knochenersatzmaterial. Im Unterschied zur Ausführungsform aus Fig. 1, ist in der Grundstruktur ein Anschlusselement 10 mit einem Verankerungsabschnitt 1 (in Fig. 2 nicht erkennbar) eingebettet. Von dem Anschlusselement in Fig. 2 ist lediglich die Aufnahmeöffnung 12 zu sehen, welche leicht über die Grundstruktur 2 des Körpers 1 hervorragt. Die Grundstruktur 2 ist als zylindrischer Körper dargestellt, kann aber je nach der Form der Kavität, in welche der Körper eingesetzt werden soll, auch andere Formen aufweisen. Zudem kann die Grundstruktur aus einem formstabilen aber leicht bearbeitbarem Material gefertigt sein, so dass er vor dem Einsetzen leicht an die Form der Kavität angepasst werden kann.

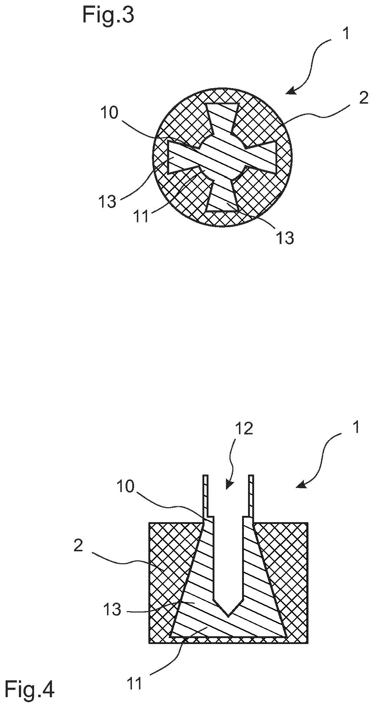

Fig. 3 zeigt einen Schnitt entlang einer Linie A-A, wie in Fig. 2 dargestellt, durch eine Ausführungsform eines erfindungsgemässen Körpers 1 mit eingebettetem Anschlusselement 10. Die gestrichelte Linie stellt die Aufnahmeöffnung 12 dar. Das Anschlusselement 10 weist im Verankerungsabschnitt 11 radial nach aussen gerichtete Flügel 13 auf, welche sich nach aussen hin verbreitern. Auf diese Weise ist das Anschlusselement 10 verdrehsicher in der Grundstruktur 2 des Körpers 1 eingebettet.

Fig.4 zeigt einen Schnitt entlang einer Linie B-B, wie in Fig. 2 dargestellt, durch eine Ausführungsform des erfindungsgemässen Körpers 1 aus Fig. 3 mit eingebettetem Anschlusselement 10. Die nach aussen gerichteten Flügel 13 verbreitern sich von der Aufnahmeöffnung weg nach unten hin. Von unten, also in Richtung der Aufnahmeöffnung, gesehen, weist der Verankerungsabschnitt 11 des Anschlusselements 10 einen Hinterschnitt auf, welcher in die Grundstruktur 2 des Körpers 1 in Eingriff ist, so dass das Anschlusselement 10 nicht ohne Beschädigung der Grundstruktur 2 aus dieser herausgezogen werden kann.

Die Grundstruktur 2 weist Hohlräume oder Poren auf, was durch die kreuzgitterartige Schraffierung in den Fig. 1 bis 4 dargestellt ist. Vorzugsweise weist die Grundstruktur eine makroporöse, interkonektierte Struktur auf. Die Grundstruktur kann z.B. eine Gyroid- oder gyroid-ähnliche Struktur aufweisen. Diese Hohlräume können mit osteoinduktiven Zusammensetzungen und/oder pharmazeutischen oder bioaktiven Produkten gefüllt resp. angereichert sein.

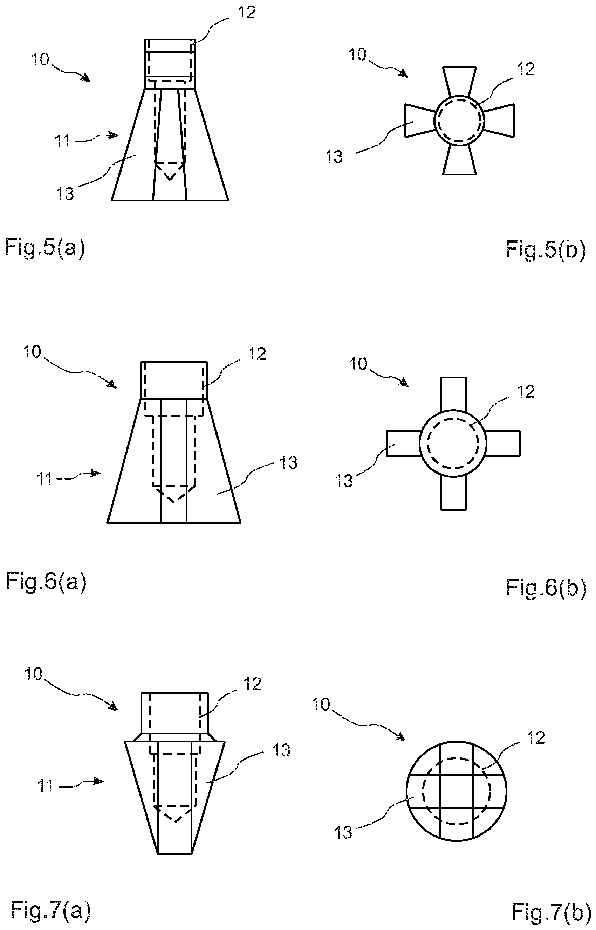

In den Figuren 5 bis 7 sind drei unterschiedliche Ausführungsformen eines Anschlusselements 10 gezeigt, unter (a) in einer Seitenansicht und unter (b) in einer Unteransicht. Die gestrichelte Linie zeigt jeweils eine Aufnahmeöffnung 12 für Verbindungs- oder Abschlusselemente. Die drei Anschlusselemente unterscheiden sich jeweils in der Ausgestaltung der Verankerungsabschnitte 11. Die Ausführungsform in Fig. 5 weist einen Verankerungsabschnitt mit vier seitlichen Flügel 13 auf, welche sich radial nach aussen verbreitern. Im Unterschied dazu sind die Flügel der Ausführungsformen in den Fig. 6 und 6 jeweils nach aussen hin gleich breit. Bei allen drei Ausführungsformen bewirken die Flügel 13 eine verdrehsichere Verankerung in einer Grundstruktur 2 eines erfindungsgemässen Körpers 1. Ein so in die Grundstruktur 2 eingebettetes Anschlusselement kann nicht aus der Grundstruktur 2 gedreht werden, ohne diese zu beschädigen.

Zudem weisen die in den Figuren 5 bis 7 gezeigten Ausführungsformen von unten in Richtung zur Aufnahmeöffnung 12 hin gesehen im Verankerungsabschnitt 11 einen Hinterschnitt auf, so dass ein in die Grundstruktur 2 eingebettetes Anschlusselement mit dem Hinterschnitt in die Grundstruktur 2 eingreift. In den Ausführungsformen in Fig. 5 und 5 wird dies erreicht, indem die Breite der Flügel 13 von der Aufnahmeöffnung weg nach unten hin zunimmt. Bei der Ausführungsform in Fig. 7 sind die Flügel genau umgekehrt geformt, so dass die Breite der Flügel 13 von der Aufnahmeöffnung 12 weg nach unten hin abnimmt, wobei diese vollständig in die Grundstruktur eingebettet werden. Auf diese Weise eingebettete Anschlusselemente können nicht aus der Grundstruktur 2 gezogen werden, ohne diese zu beschädigen.

Andere Formen der Verankerung, insbesondere einer verdrehsicheren Verankerung, sind auch möglich. Z.B. kann der Verankerungsabschnitt einen eckigen, insbesondere rechteckigen, Querschnitt aufweisen. Anstelle der Flügel können auch Elemente wie Rippen, Flossen, Noppen, Haken, Vorsprünge, angeformte Plättchen, Gitter oder dergleichen ausgebildet sein. Zudem kann die Anzahl dieser Element variieren.

Auch können die vorgenannten Hinterschnitt z.B. durch unterschiedlich grosse Querschnitte des Verankerungselements erreicht werden oder durch Aussparungen im Anschlusselement, in welche die Grundstruktur eingreifen kann.

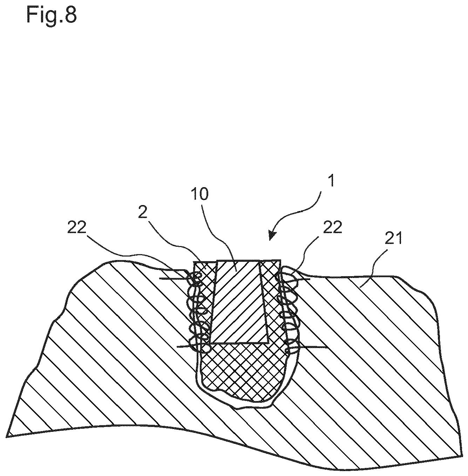

Fig. 8 zeigt eine schematische Darstellung eines erfindungsgemässen Körpers 1 mit eingebettetem Anschlusselement 10. Wie auch bei den anderen Ausführungsformen aus den Figuren 1 bis 4 weist die Grundstruktur 2 des Körpers 1 eine grobporöse, interkonektierte Struktur auf. Diese Struktur kann dazu dienen, den Körper 1 mittels einem oder mehreren Fäden 22 an dem Knochen 22 festzunähen.

### BEZEICHNUNGSLISTE

- 1: Körper
- 2: Grundstruktur
- 10: Anschlusselement
- 11: Verankerungsabschnitt
- 12: Aufnahmeöffnung
- 13: Flügel
- 14: Kopf
- 15: Hals
- 21: Knochen
- 22: Faden

## Patentansprüche

1. Biokompatibler Körper (1) zur Implantation in eine Kavität eines Knochens, umfassend eine Grundstruktur (2) aus einem Knochenersatzmaterial, wobei die Grundstruktur (2) mehrere übereinander liegende Schichten umfasst, wobei die Schichten jeweils mehrer beabstandete, vorzugsweise parallel zueinander verlaufende Linien aus Knochenersatzmaterial umfassen, **dadurch gekennzeichnet, dass** der Körper (1) ein Anschlusselement (10) für ein Abutment, eine Krone, eine Brücke oder eine andere Prothetik aufweist, wobei das Anschlusselement (10) zumindest teilweise in der Grundstruktur (2) des Körpers (1) eingebettet ist, wobei das Anschlusselement (10) eine Aufnahmeöffnung (12) für ein Verbindungs- oder Abschlusselement aufweist und die Aufnahmeöffnung (12) nach dem Einsetzen des biokompatiblen Körpers (1) in die Kavität des Knochens von ausserhalb der Grundstruktur (2) des Körpers (1) für das Verbindungs- oder Abschlusselement zugänglich ist, und wobei das Anschlusselement (10) einen Verankerungsabschnitt (11) aufweist, wobei das Anschlusselement (10) mit den Verankerungsabschnitt formschlüssig und verdrehsicher in der Grundstruktur (2) des Körpers (1) eingebettet ist.

2. Biokompatibler Körper (1) nach Anspruch 1, **dadurch gekennzeichnet, dass** das Anschlussstück einen Kopf aufweist, welcher derart in die Grundstruktur des Körpers eingebettet ist, dass die Grundstruktur den Kopf des Anschlussstücks kragenförmig umschliesst.

3. Biokompatibler Körper (1) nach einem der Ansprüche 1 bis 2, **dadurch gekennzeichnet, dass** der Verankerungsabschnitt (11) des Anschlusselements einen Hinterschnitt aufweist, mit welchem das Anschlusselement in der Grundstruktur in Eingriff ist.

4. Biokompatibler Körper (1) nach einem der vorangehenden Ansprüche, **dadurch gekennzeichnet, dass** das Anschlusselement einstückig ausgebildet ist.

5. Biokompatibler Körper (1) nach einem der vorangehenden Ansprüche, **dadurch gekennzeichnet, dass** die Grundstruktur aus Knochenersatzmaterial osteokonduktiv ist.

6. Biokompatibler Körper (1) nach einem der vorangehenden Ansprüche, **dadurch gekennzeichnet, dass** die Grundstruktur aus Knochenersatzmaterial Höhlräume aufweist, welche vorzugsweise mit einer osteoinduktiven Zusammensetzung und/oder pharmazeutischen oder bioaktiven Produkten angereichert sind.

7. Biokompatibler Körper (1) nach einem der vorangehenden Ansprüche, **dadurch gekennzeichnet, dass** die Grundstruktur aus Knochenersatzmaterial eine makroporöse, interkonektierte Struktur, vorzugsweise eine Gyroid- oder gyroid-ähnliche Struktur, aufweist.

8. Biokompatibler Körper (1) nach einem der vorangehenden Ansprüche, **dadurch gekennzeichnet, dass** er eine zweite Grundstruktur aus einem biokompatiblen Material, vorzugsweise ein Knorpelersatzmaterial oder ein Ersatz-Weichgewebe, z.B. ein Ersatz-Hautgewebe, aufweist.

9. Verfahren zur Herstellung eines biokompatiblen Körpers (1) nach einem der vorangehenden Ansprüche, umfassend die folgenden Schritte:
- Bereitstellen eines Anschlusselements;
Anbringen einer Grundstruktur aus Knochenersatzmaterial, so dass das Anschlusselement in der Grundstruktur aus Knochenersatzmaterial eingebettet ist.

10. Verfahren zur Herstellung eines biokompatiblen Körpers (1) nach Anspruch 9, **dadurch gekennzeichnet, dass** das Anbringen der Grundstruktur die folgenden Schritte umfasst:
a) Anbringen einer Schicht der Grundstruktur aus Knochenersatzmaterial in voneinander beabstandeten, vorzugsweise parallelen, Linien, so dass Zwischenräume entstehen;
b) Anreichern der Zwischenräume in der Schicht mit osteoinduktiven Zusammensetzungen;
c) Wiederholen der Schritte a) und b) bis eine mehrschichtige Grundstruktur entstanden ist, welche das Anschlusselement zumindest teilweise einbettet.

11. Verfahren nach Anspruch 10, **dadurch gekennzeichnet, dass** beim Schritt c) die Linien der anzubringenden Schicht in einem Winkel, vorzugsweise von etwa 30 bis 90 Grad, zu den Linien der vorangehenden Schicht stehen, so dass eine makroporöse interkonektierte Struktur entsteht.

12. Verfahren nach einem der Ansprüche 10 bis 11, **dadurch gekennzeichnet, dass** gleichzeitig mit oder nach dem Anbringen der Grundstruktur aus Knochenersatzmaterial eine zweite Grundstruktur aus einem biokompatiblen Material, vorzugsweise Knorpelersatzmaterial oder Ersatz-Weichgewebe, z.B. ein Ersatz-Hautgewebe, angebracht wird.

## Claims

1. A biocompatible body (1) for implantation into a cavity of a bone, comprising a main structure (2) formed from a bone substitute material, wherein the main structure (2) comprises a plurality of superimposed layers, wherein the layers respectively comprise a plurality of lines which spaced apart from each other and are preferably parallel to each other, formed from bone substitute material, **characterized in that** the body (1) has a connector element (10) for an abutment, a crown, a bridge or another prosthesis, wherein the connector element (10) is at least partially embedded in the main structure (2) of the body (1), wherein the connector element (10) has a receiving opening (12) for a connecting or terminating element and the receiving opening (12) for the connecting or terminating element is accessible from outside the main structure (2) of the body (1) after insertion of the biocompatible body (1) into the cavity of the bone, and wherein the connector element (10) is provided with an anchoring segment (11), wherein the connector element (10) is with its the anchoring segment embedded in the main structure (2) of the body (1) in a positive locking manner and secured against twisting.

2. The biocompatible body (1) as claimed in claim 1, **characterized in that** the connecting piece has a head which is embedded in the main structure of the body in a manner such that the main structure encloses the head of the connecting piece in the manner of a collar.

3. The biocompatible body (1) as claimed in claim 1 or claim 2, **characterized in that** the anchoring segment (11) of the connector element is provided with an undercut by means of which the connector element engages with the main structure.

4. The biocompatible body (1) as claimed in one of the preceding claims, **characterized in that** the connector element is formed as a single piece.

5. The biocompatible body (1) as claimed in one of the preceding claims, **characterized in that** the main structure formed from bone substitute material is osteoconductive.

6. The biocompatible body (1) as claimed in one of the preceding claims, **characterized in that** the main structure formed from bone substitute material is provided with cavities which are preferably fortified with an osteoinductive composition and/or pharmaceutical or bioactive products.

7. The biocompatible body (1) as claimed in one of the preceding claims, **characterized in that** the main structure formed from bone substitute material has a macroporous, interconnected structure, preferably a gyroid or gyroid-like structure.

8. The biocompatible body (1) as claimed in one of the preceding claims, **characterized in that** it has a second main structure produced from a biocompatible material, preferably a cartilage substitute material or soft tissue substitute, for example a skin tissue substitute.

9. A method for the production of a biocompatible body (1) as claimed in one of the preceding claims, comprising the following steps:
- preparing a connector element;
positioning a main structure formed from bone substitute material, so that the connector element is embedded in the main structure formed from bone substitute material.

10. The method for the production of a biocompatible body (1) as claimed in claim 9, **characterized in that** positioning of the main structure comprises the following steps:
a) depositing a layer of the main structure formed from bone substitute material in lines spaced apart from each other and preferably parallel, so that interstices are formed;
b) fortifying the interstices in the layer with osteoinductive compositions;
c) repeating steps a) and b) until a multi-layered main structure has been formed which at least partly embeds the connector element.

11. The method as claimed in claim 10, **characterized in that** in step c), the lines of the layer being deposited make an angle of approximately 30 to 90 degrees with the lines of the previous layer, so that a macroporous interconnected structure is formed.

12. The method as claimed in claim 10 or claim 11, **characterized in that** a second main structure of biocompatible material, preferably cartilage substitute material or soft tissue substitute, for example a skin tissue substitute, is deposited at the same time or after depositing the main structure formed from bone substitute material.

## Revendications

1. Corps biocompatible (1) destiné à l'implantation dans une cavité d'un os, comprenant une structure de base (2) dans un matériau de remplacement osseux, dans lequel la structure de base (2) comprend plusieurs couches reposant l'une sur l'autre, dans lequel les couches comprennent respectivement plusieurs lignes en matériau de remplacement osseux espacées, de préférence parallèles entre elles, **caractérisé en ce que** le corps (1) présente un élément de raccord (10) pour un appui, une couronne, un pont ou une autre prothétique, dans lequel l'élément de raccord (10) est enchâssé au moins partiellement dans la structure de base (2) du corps (1), dans lequel l'élément de raccord (10) présente une ouverture de réception (12) pour un élément de liaison ou de raccord et l'ouverture de réception (12) est accessible après l'insertion du corps biocompatible (1) dans la cavité de l'os depuis l'extérieur de la structure de base (2) du corps (1) pour l'élément de liaison ou de raccord, et dans lequel l'élément de raccord (10) présente un tronçon d'ancrage (11), dans lequel l'élément de raccord (10) est enchâssé dans la structure de base (2) du corps (1) par complémentarité de forme et résistant à la torsion par le tronçon d'ancrage.

2. Corps biocompatible (1) selon la revendication 1, **caractérisé en ce que** la pièce de raccord présente une tête qui est ainsi enchâssée dans la structure de base du corps que la structure de base entoure la tête de la pièce de raccord en formant un col.

3. Corps biocompatible (1) selon l'une des revendications 1 à 2, **caractérisé en ce que** le tronçon d'ancrage (11) de l'élément de raccord présente une contre-dépouille avec laquelle l'élément de raccord est en prise dans la structure de base.

4. Corps biocompatible (1) selon l'une des revendications précédentes, **caractérisé en ce que** l'élément de raccord est conçu d'une seule pièce.

5. Corps biocompatible (1) selon l'une des revendications précédentes, **caractérisé en ce que** la structure de base en matériau de remplacement osseux est ostéoconductrice.

6. Corps biocompatible (1) selon l'une des revendications précédentes, **caractérisé en ce que** la structure de base en matériau de remplacement osseux présente des cavités qui sont enrichies de préférence avec une composition ostéoinductrice et/ou des produits pharmaceutiques ou bio-actifs.

7. Corps biocompatible (1) selon l'une des revendications précédentes, **caractérisé en ce que** la structure de base en matériau de remplacement osseux présente une structure macro-poreuse, interconnectée, de préférence une structure de gyroïde ou similaire à un gyroïde.

8. Corps biocompatible (1) selon l'une des revendications précédentes, **caractérisé en ce qu'**il présente une seconde structure de base dans un matériau biocompatible, de préférence un matériau de remplacement de cartilage ou un tissu mou de remplacement, par exemple un tissu cutané de remplacement.

9. Procédé de fabrication d'un corps biocompatible (1) selon l'une des revendications précédentes, comprenant les étapes suivantes:
- préparation d'un élément de raccord ;
application d'une structure de base en matériau de remplacement osseux, de sorte que l'élément de raccord soit enchâssé dans la structure de base en matériau de remplacement osseux.

10. Procédé de fabrication d'un corps biocompatible (1) selon la revendication 9, **caractérisé en ce que** l'application de la structure de base comprend les étapes suivantes :
a) application d'une couche de la structure de base en matériau de remplacement osseux dans des lignes espacées, de préférence parallèles entre elles, de façon à produire des espaces intermédiaires ;
b) enrichissement des espaces intermédiaires dans la couche par des compositions ostéoinductrices ;
c) répétition des étapes a) et b) jusqu'à ce qu'une structure de base multi-couches soit produite qui enchâsse au moins partiellement l'élément de raccord.

11. Procédé selon la revendication 10, **caractérisé en ce qu'**à l'étape c), les lignes de la couche à appliquer sont dans un angle de préférence d'environ 30 à 90 degrés, par rapport aux lignes de la couche précédente, de façon à ce qu'une structure interconnectée macro-poreuse soit produite.

12. Procédé selon l'une des revendications 10 à 11, **caractérisé en ce que** parallèlement à ou après l'application de la couche de base en matériau de remplacement osseux, une seconde structure en matériau biocompatible, de préférence un matériau de remplacement de cartilage ou un tissu mou de remplacement, par exemple un tissu cutané de remplacement, est appliquée.
